# FASCICULE DE BREVET EUROPEEN

(11) **EP 1 673 122 B1**
(45) Date de publication et mention de la délivrance du brevet: **16.08.2017**
(21) Numéro de dépôt: 04787376.5
(22) Date de dépôt: 14.09.2004
(51) Int. Cl.: A61M 5/20, A61M 5/24, A61M 5/32

(54) **DISPOSITIF D`INJECTION D`UN PRODUIT, NOTAMMENT A USAGE MEDICAL**
VORRICHTUNG ZUR INJEKTION EINES PRODUKTS, PRIMÄR FÜR DIE MEDIZINISCHE ANWENDUNG
DEVICE FOR INJECTING A PRODUCT, PRIMARILY FOR MEDICAL USE

(30) Priorité: 26.09.2003 FR 0311314
(43) Date de publication de la demande: 28.06.2006
(73) Titulaire: Becton Dickinson France, 38800 Le Pont-de-Claix (FR)
(72) Inventeur: VEDRINE, Lionel, Palo Alto, CA 94306 (US)
(74) Mandataire: Brédeville, Odile Marie
(86) Numéro de dépôt international: PCT/FR2004/002330
(87) Numéro de publication internationale: WO 2005/030302

(56) Documents cités:
- EP-A- 0 516 473
- WO-A-01/07104
- FR-A- 2 842 428
- US-A- 2 752 918

## Description

La présente invention concerne un dispositif d'injection d'un produit, notamment à usage médical. Ce dispositif est notamment destiné à permettre de réaliser une injection intra-dermale.

Dans la description ci-après, les termes "proximal " et "distal" sont considérés par rapport au sens d'injection du produit.

Il est courant de réaliser une injection intra-dermale au moyen d'une seringue classique, en piquant selon une direction formant un angle faible avec la peau.

Ces seringues classiques n'assurent pas une parfaite fiabilité de l'injection ni une parfaite sécurité contre les risques de piqûres accidentelles susceptibles de se produire après l'injection.

Afin de réduire au maximum ces risques, il est important de limiter la liberté d'action de l'utilisateur final de tels dispositifs d'injection.

L'invention vise à remédier à cet inconvénient fondamental.

Il est par ailleurs connu du document WO0107104 un dispositif d'injection automatique de liquides à injecter. Le document EP0516473 divulgue un dispositif d'injection automatique. Il est connu du document US2752918 une seringue hypodermique automatique et son ampoule. En outre, le document FR2842428 divulgue un dispositif d'injection d'un produit, notamment à usage médical.

L'objectif de l'invention est donc de fournir un dispositif assurant une parfaite fiabilité de l'injection et une parfaite sécurité contre les risques de piqûres accidentelles en limitant au maximum les actions de l'utilisateur final.

La présente invention porte sur un dispositif d'injection d'un produit, notamment à usage médical, selon la revendication 1.

Avant injection, le support est maintenu en position d'attente fixe par rapport au corps. Le récipient est alors relié au support. L'utilisateur agit sur les moyens d'activation du relâchement des moyens de maintien du support. Le déplacement du support par rapport au corps dans le sens distal entraîne le déplacement du récipient dans le même sens. L'aiguille étant par ailleurs maintenue par rapport au corps en position d'injection, le mouvement du récipient génère une force sur le piston qui fournit une pression sur le liquide. Cette pression pousse le liquide contenu dans le récipient vers l'aiguille et la peau du patient.

Les premiers moyens d'actionnement sont distincts des seconds moyens d'actionnement. Ainsi, en fin d'injection, lesdits premiers moyens d'actionnement provoquent le relâchement desdits moyens de maintien de l'aiguille tandis que, soit simultanément soit ultérieurement, les seconds moyens d'actionnement provoquent le relâchement desdits moyens de maintien du récipient, ce qui permet d'amener l'aiguille et le récipient en position de rétraction. Cette rétraction permet d'assurer une parfaite sécurité contre les risques de piqûres accidentelles.

En particulier, lorsque les seconds moyens d'actionnement provoquent le relâchement desdits moyens de maintien du récipient ultérieurement au relâchement desdits moyens de maintien de l'aiguille par les premiers moyens d'actionnement, alors le risque de rentrer l'aiguille avant que la dose complète soit administrée (fuite, dose déficiente, etc...) est réduit.

Avantageusement, les moyens de maintien du support comprennent :
- une bague, montée de façon fixe à l'intérieur du corps et dans la partie proximale de ce dernier, cette bague comprenant à son extrémité distale au moins une dent transversale,
- au moins un crochet situé à l'extrémité proximale du support et destiné à s'encliqueter dans ladite dent,
- un ressort dont l'extrémité distale prend appui sur un décrochement interne transversal de l'extrémité distale du support et dont l'extrémité proximale prend appui sur une paroi transversale située à l'extrémité distale de la bague, ledit ressort étant à l'état comprimé lorsque la dent est encliquetée dans le crochet.

Avantageusement, les moyens d'activation du relâchement des moyens de maintien du support sont sous la forme d'un bouton solidaire de la dent de la bague, ledit bouton faisant saillie à l'extérieur du corps à travers une fenêtre ménagée dans le corps à cet effet.

Ainsi, lorsque l'utilisateur final appuie sur le bouton, la dent, qui est solidaire du bouton, se défléchit dans le sens radial vers l'axe du dispositif et elle se libère du crochet du support. Le support n'étant plus retenu à la bague, le ressort se détend entraînant avec lui le support qui se déplace dans le sens distal. Le support étant par ailleurs relié au récipient, il entraîne ce dernier dans le sens distal et permet l'injection.

Le dispositif selon l'invention présente l'avantage de ne nécessiter qu'une intervention minimale de la part de l'utilisateur final. Ce dernier n'a qu'à appuyer sur le bouton de la bague pour réaliser l'injection.

Avantageusement, le dispositif comprend des moyens à ressort permettant d'amener l'aiguille et le récipient en position de rétraction sans intervention volontaire extérieure, en fin d'injection.

Selon une possibilité de mise en oeuvre de l'invention, lesdits moyens de maintien de l'aiguille comprennent :
- une pièce supportant l'aiguille, comportant au moins un moyen de verrouillage ;
- au moins une patte comportant un moyen de verrouillage propre à coopérer avec celui de ladite pièce supportant l'aiguille, cette patte étant mobile radialement entre une position radialement interne normale, dans laquelle lesdits moyens de verrouillage viennent en prise de manière à maintenir ladite pièce supportant l'aiguille par rapport audit corps, et une position radialement externe, dans laquelle une zone du support vient déplacer cette patte radialement vers l'extérieur de manière à libérer ledit verrouillage, ce qui libère par conséquent ladite pièce supportant l'aiguille par rapport audit corps.

Selon une possibilité de mise en oeuvre de l'invention, lesdits moyens de maintien du récipient comprennent :
- une collerette formée au niveau de l'extrémité du récipient opposée à l'extrémité fermée de ce récipient ;
- des moyens de prise solidaires dudit support, permettant de relier ladite collerette au support ; et
- au moins une patte comportant lesdits moyens de prise, mobile dans le sens radial de ce support, entre une position radialement interne, dans laquelle lesdits moyens de prise relient ladite collerette au support, et une position radialement externe, dans laquelle lesdits moyens de prise sont effacés radialement au delà cette collerette, qu'ils libèrent par conséquent.

Le piston engagé dans le récipient est de préférence conformé pour, dans une première conformation du piston ou position relative de ce piston et de ce récipient, fermer le récipient de manière à isoler le produit par rapport à l'extérieur de ce récipient et, dans une deuxième conformation du piston ou position relative de ce piston et de ce récipient, permettre le passage du produit vers l'extérieur du récipient. Le piston peut notamment comprendre au moins une zone périphérique, propre, dans ladite première conformation du piston, à appuyer étroitement contre la paroi du récipient, et, dans ladite deuxième conformation du piston, à s'effacer sous la pression du produit à injecter pour permettre le passage de ce dernier.

Le piston peut également comprendre une zone transperçable placée en regard de l'extrémité proximale de l'aiguille. Le déplacement du récipient par rapport à l'aiguille conduit alors l'extrémité proximale de l'aiguille à transpercer cette zone transperçable du piston jusqu'à venir en communication avec le produit à injecter et permettre l'écoulement de ce produit à travers l'aiguille.

Les figures annexées illustrent, à titre d'exemple, un mode de réalisation préféré du dispositif selon l'invention.
La figure 1 en est une vue en perspective éclatée, en coupe passant par son axe ;
la figure 2 en est une vue en perspective à l'état monté ;
les figures 3 à 5 sont des vues en coupe du dispositif selon l'invention respectivement en position de stockage, en position de fin d'injection et en position de rétraction,
la figure 6 est une vue en coupe selon la ligne XX de la figure 3 du dispositif en position de stockage,
la figure 7 est une vue en coupe partielle de la position du piston en phase d'injection,
les figures 8 et 9 sont des vues en coupe selon la ligne XX de la figure 3 du dispositif en position respectivement de fin d'injection et de rétraction,
les figures 10 à 12 sont des vues de côté simplifiées, du dispositif en position respectivement de stockage, de fin d'injection et de rétraction, montrant le relâchement des moyens de maintien de l'aiguille,
la figure 13 est une vue en perspective du dispositif selon l'invention.

Les figures représentent un dispositif 1 d'injection d'un produit, notamment à usage médical.

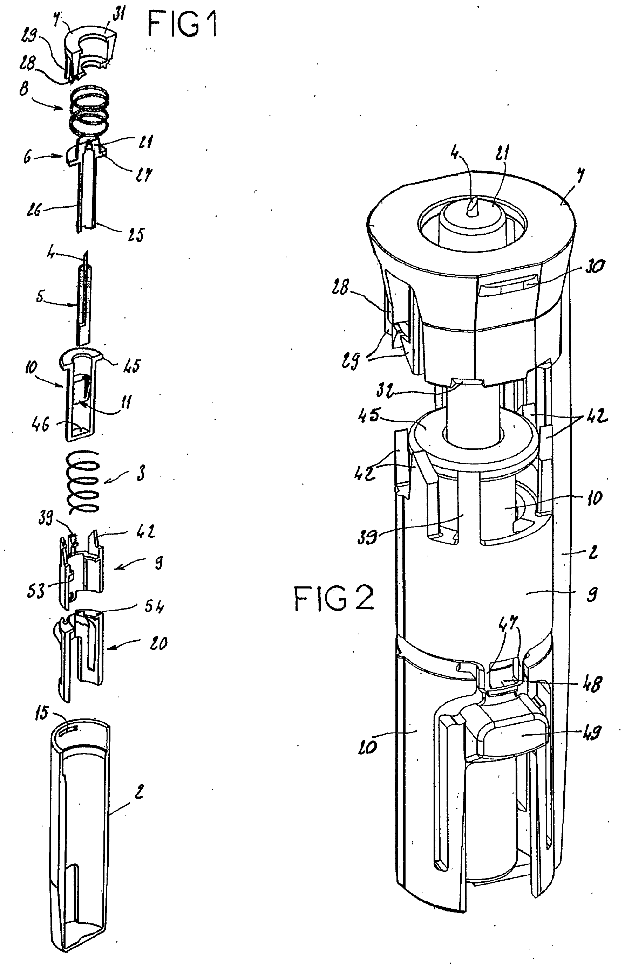

Comme le montrent plus particulièrement les figures 1 et 2, le dispositif 1 comprend un corps 2, une aiguille creuse d'injection 4, un ressort d'activation d'injection 3, des pièces 5 à 7 de montage de l'aiguille 4, un ressort d'activation de rétraction 8, un support de récipient 9, un récipient 10, un piston 11 et une bague 20, décrits en détails ci-après.

Le corps 2 présente une forme générale tubulaire et comprend une nervure circulaire 15 au niveau de son extrémité distale.

L'aiguille 4 est fixée à la pièce 5. Celle-ci a une forme pleine généralement cylindrique, et présente une rainure et un perçage qui forment un conduit d'écoulement communiquant avec la cavité de l'aiguille 4.

La pièce 6 présente une partie proximale 26 de forme tubulaire qui reçoit étroitement la pièce 5 en elle, et comprend un trou distal pour permettre l'engagement de l'aiguille 4 au travers d'un bossage 21. La face distale du bossage 21 forme une surface d'engagement du dispositif 1 avec la peau du patient. De préférence, en position d'injection, l'aiguille 4 dépasse de la surface d'engagement du dispositif 1 avec la peau du patient sur une distance allant de 0,5 mm à 3 mm. La partie 26 est destinée à être introduite dans le récipient 10, comme mentionné plus haut et comporte un joint d'étanchéité 25 à son niveau proximal. Cette partie 26 permet ainsi de déplacer le piston 11 dans le récipient 10 lorsque le support 9 est déplacé par rapport au corps 2, comme cela apparaîtra plus loin.

La pièce 6 comprend également une collerette 27 propre à être encliquetée, au moyen d'un ergot 27a, dans des ouvertures que présentent quatre pattes 29 solidaires de la pièce 7, lesdites pattes 29 étant disposées deux à deux en regard d'une paroi 28 s'étendant dans le sens proximal à partir de l'extrémité distale de la pièce 7, lesdites pattes 29 étant aptes à se défléchir circonférentiellement.

La pièce 7 est destinée à être insérée étroitement dans l'ouverture distale du corps 2, une collerette distale 30 qu'elle comprend prenant place dans l'évidement distal délimité par la nervure 15. Cet engagement étroit permet la fixation de la pièce 7 au corps 2.

La pièce 7 comprend également une ouverture délimitée par un rebord 31 de diamètre inférieur au diamètre du ressort d'activation de rétraction 8.

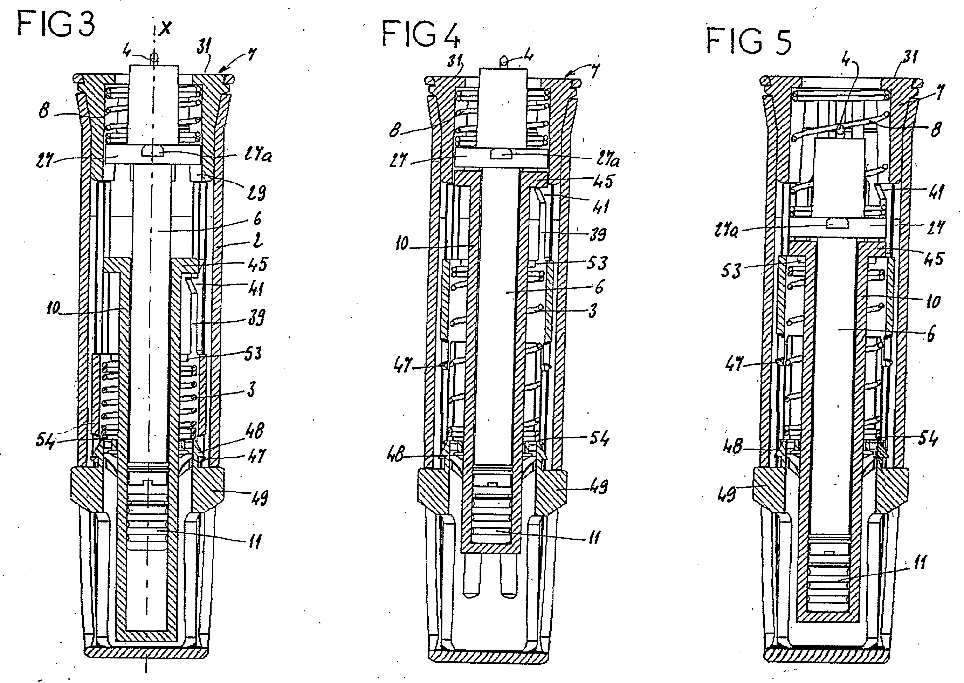

Comme le montre la figure 3, ce rebord 31 permet le maintien du ressort 8 à l'état comprimé entre la face proximale de ce rebord 31 et la face distale de la collerette 27 lorsque la pièce 6 est encliquetée dans la pièce 7.

La pièce 7 comprend en outre, à son extrémité proximale, deux chanfreins 32 formant des rampes. Il apparaît sur la figure 2 que les pattes 29 présentent des rampes inclinées internes aménagées dans leurs parties proximales.

Le support 9 est engagé dans le corps 2 et peut coulisser par rapport à celui-ci.

À son niveau distal, le support 9 forme deux pattes 39 mobiles radialement, pourvues de saillies internes 41 (voir figure 3) formant des butées de réception d'une collerette 45 que comprend le récipient 10. Cette venue en butée de la collerette 45 contre les saillies 41 permet de lier la collerette 45 au support 9 dans le sens de déplacement du support 9 qui permet de réaliser l'injection.

Le support 9 forme également quatre parois 42 situées entre les pattes 39. Comme le montrent les figures 1 à 3, les pattes 39 comprennent, au niveau de leurs extrémités distales, des rampes inclinées internes propres à venir coopérer avec les rampes des chanfreins 32 en fin de course d'injection, et les parois 42 comprennent, au niveau de leurs extrémités distales, des rampes inclinées externes propres à venir coopérer avec les rampes internes des pattes 29 également en fin de course d'injection.

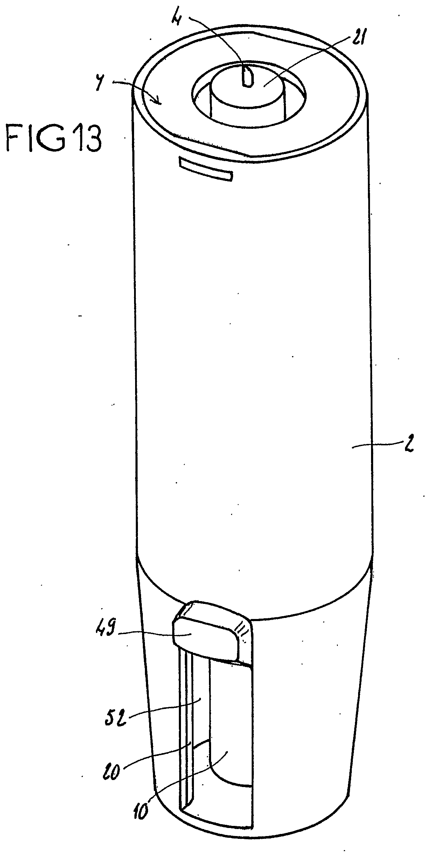

A son extrémité proximale, le support 9 comprend au moins un crochet 47 destiné à s'encliqueter avec une dent 48 transversale située sur l'extrémité distale de la bague 20. Cette bague 20 est montée de façon fixe à l'intérieur du corps 2 dans la partie proximale de ce dernier. La bague 20 comprend également sur sa face externe au moins un bouton 49 solidaire de la dent 48 et faisant saillie à l'extérieur du corps 2 à travers une fenêtre 52 ménagée dans le corps 2 à cet effet comme montré sur la figure 13.

Un ressort d'activation d'injection 3 est disposé entre le support 9 et la bague 20. Comme montré sur la figure 3, l'extrémité distale de ce ressort 3 prend appui sur un décrochement interne transversal 53 de l'extrémité distale du support 9 et son extrémité proximale prend appui sur une paroi transversale 54 située à l'extrémité distale de la bague 20. Le ressort d'activation d'injection 3 est à l'état comprimé lorsque la dent 48 est encliquetée dans le crochet 47 comme montré sur la figure 3.

Du côté opposé à la collerette 45, le récipient 10 comprend un fond 46. Le produit à injecter est contenu entre le piston 11 et les parois du récipient 10.

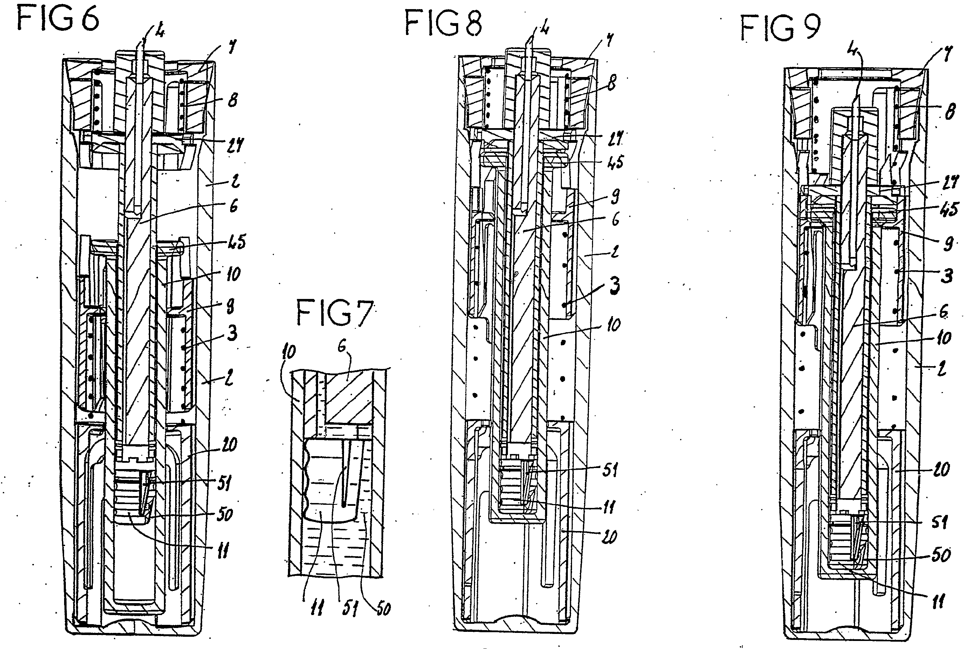

Le piston 11 est en une matière souple, notamment en élastomère. Il présente une forme conique et est placé dans le récipient 10 de telle sorte que sa face de plus faible surface soit tournée vers le produit à injecter. Il ménage ainsi, comme le montre la figure 6, un interstice 50 entre lui et la paroi du récipient 11. De plus, le piston 11 comprend un trou borgne latéral 51 aménagé sur une majeure partie de son épaisseur, à partir de sa face axiale distale, du côté de la paroi latérale du piston 11 qui permet de délimiter ledit interstice 50. Le trou 51 a une forme telle qu'il suit, au moins approximativement, cette paroi latérale, et délimite ainsi une zone périphérique s'étendant sur une portion de la périphérie du piston 11.

Comme le montre la comparaison des figures 6 et 7, cette zone périphérique adopte normalement une position radialement externe montrée sur la figure 6, dans laquelle elle appuie étroitement contre la paroi du récipient 10, et peut prendre une position radialement interne montrée sur la figure 7, dans laquelle elle s'efface sous la pression du produit à injecter lors du passage de ce dernier entre le piston 11 et le récipient 10, résultant de l'appui du piston 11 contre le produit.

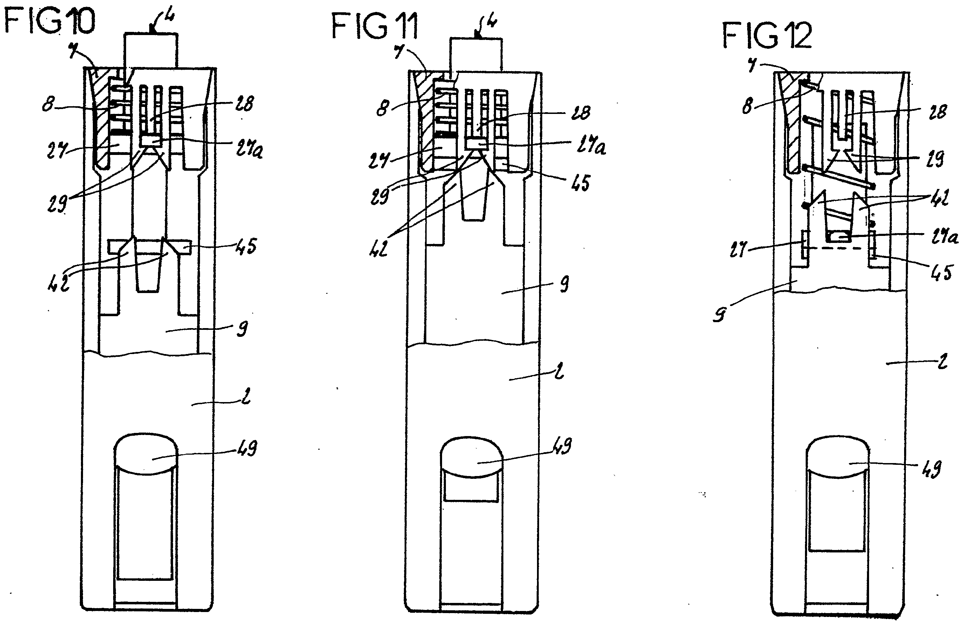

En pratique, le dispositif 1 se trouve à l'origine dans la position de stockage représentée sur les figures 3, 6 et 10, dans laquelle l'ergot 27a de la collerette 27 est en prise avec les pattes 29 et la collerette 45 est maintenue par les saillies 41. Dans cette position, l'aiguille 4 fait saillie au-delà de l'extrémité distale du dispositif selon la profondeur recherchée pour l'injection, qui est une injection intra-dermale dans l'exemple représenté. Dans cette position également, la dent 48 est encliquetée dans le crochet 47 et le ressort d'activation d'injection 3 est à l'état comprimé.

L'utilisateur final appuie sur le ou les boutons 49 faisant saillie du corps 2 par la fenêtre 52. Le bouton 49 étant solidaire de la dent 48, celle-ci se défléchit radialement vers l'axe du dispositif sous la pression exercée sur le bouton 49. La dent 48 se libère alors du crochet 47 et le ressort d'activation d'injection 3 se détend, entraînant avec lui le support 9 dans le sens distal. Le support 9 étant relié au récipient 10 du fait que la collerette 45 du récipient 10 est maintenue par les pattes 39 du support 9, le récipient 10 se déplace également dans le sens distal.

Le déplacement du récipient 10 avec le support 9 presse le piston 11 contre le produit à injecter, ce qui amène à l'écoulement du produit entre le piston 11 et le récipient 10, comme cela apparaît sur la figure 7.

Comme montré aux figures 4, 8 et 11, à l'approche de la position de fin de course d'injection, les rampes des pattes 39 et des parois 42 viennent porter contre, respectivement, les rampes des chanfreins 32 et des pattes 29, de telle sorte que les pattes 29 sont défléchies circonférentiellement et les pattes 39 sont déplacées vers des positions radialement extérieures, positions dans lesquelles les pattes 29 et 39 libèrent respectivement l'ergot 27a de la collerette 27 et la collerette 45. Le ressort d'activation de rétraction 8 peut alors se relâcher, ce qui provoque un recul simultané des pièces 5 et 6, et donc de l'aiguille 4, ainsi que du récipient 10 du fait du frottement du joint 25, vers une position de rétraction montrée sur les figures 5, 9 et 12. Dans cette position, l'extrémité distale de l'aiguille 4 se trouve en deçà de la face distale de la pièce 7 et la collerette 45 se trouve en deçà, du côté proximal, des saillies 41.

Il apparaît de ce qui précède que l'invention apporte des améliorations déterminantes aux dispositifs homologues de la technique antérieure, en ne nécessitant de la part de l'utilisateur final qu'une intervention minimale pour réaliser à la fois l'injection et la rétraction de l'aiguille, assurant ainsi une parfaite sécurité contre les risques de piqûres accidentelles susceptibles de se produire après l'injection.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. En particulier, le piston peut comprendre une zone transperçable placée en regard de l'extrémité proximale de l'aiguille, cette extrémité proximale dépassant, du côté proximale de la pièce 5 qui le comporte.

## Revendications

1. Dispositif (1) d'injection d'un produit, notamment à usage médical, comprenant:
- un corps (2) recevant une aiguille (4) creuse d'injection et un récipient (10) contenant le produit à injecter; l'aiguille (4) et le récipient (10) étant mobiles par rapport audit corps entre une position d'injection et une position de rétraction;
- des moyens de maintien de l'aiguille (5 à 7; 28, 29), maintenant normalement l'aiguille (4) en position d'injection, qui peuvent être relâchés pour libérer le déplacement de l'aiguille (4) vers ladite position de rétraction;
- des moyens de maintien (39, 41, 45) du récipient (10), maintenant normalement le récipient (10) en position permettant l'injection, qui peuvent être relâchés pour libérer le déplacement du récipient (10) vers ladite position de rétraction; et
- un piston (11) engagé dans le récipient (10),
- des premiers moyens (42) d'actionnement desdits moyens (5 à 7 ; 28,29) de maintien de l'aiguille (4),
- des seconds moyens (32) d'actionnement desdits moyens (39, 41, 45) de maintien du récipient (10),
- un support (9) de récipient monté coulissant sur le corps (2) et déplaçable par rapport à celui-ci pour réaliser l'injection, ledit récipient (10) étant relié à ce support (9) en étant mobile par rapport à celui-ci entre une position permettant l'injection et une position de rétraction,
- des moyens de maintien (3, 47, 48) du support (9) de récipient, maintenant normalement le support (9) de récipient en position d'attente avant l'injection, qui peuvent être relâchés pour libérer le déplacement du support (9) de récipient et permettre l'injection,
- des moyens d'actionnement (49) du relâchement des moyens de maintien (3, 20, 47, 48) du support (9) récipient permettant l'injection,
**caractérisé en ce que** :
- lesdits premiers moyens d'actionnement sont distincts desdits seconds moyens d'actionnement, lesdits premiers et seconds moyens d'actionnement (42, 32) permettant, en fin d'injection, de respectivement relâcher les moyens (5 à 7 ; 28, 29) de maintien de l'aiguille (4) avant le relâchement des moyens (39, 41, 45) de maintien du récipient (10), ou de simultanément relâcher les moyens (5 à 7 ; 28, 29) de maintien de l'aiguille (4) et les moyens (39, 41, 45) de maintien du récipient (10).

2. Dispositif (1) selon la revendication 1, **caractérisé en ce que** les moyens de maintien (3, 20, 47, 48) du support comprennent:
- une bague (20), montée de façon fixe à l'intérieur du corps (2) et dans la partie proximale de ce dernier, cette bague (20) comprenant à son extrémité distale au moins une dent (48) transversale,
- au moins un crochet (47) situé à l'extrémité proximale du support (9) et destiné à s'encliqueter dans ladite dent (48),
- un ressort d'activation d'injection (3) dont l'extrémité distale prend appui sur un décrochement interne transversal (53) de l'extrémité distale du support (9) et dont l'extrémité proximale prend appui sur une paroi transversale (54) située à l'extrémité distale de la bague (20), ledit ressort (3) étant à l'état comprimé lorsque la dent (48) est encliquetée dans le crochet (47).

3. Dispositif (1) selon la revendication 2, **caractérisé en ce que** les moyens d'activation (49) du relâchement des moyens de maintien du support (9) sont sous la forme d'un bouton (49) solidaire de la dent (48) de la bague (20), ledit bouton (49) faisant saillie à l'extérieur du corps (2) à travers une fenêtre (52) ménagée dans le corps (2) à cet effet.

4. Dispositif (1) selon l'une quelconque des revendications précédentes, **caractérisé en ce que**, en position d'injection, l'aiguille (4) dépasse d'une surface d'engagement du dispositif (1) avec la peau du patient, sur une distance allant de 0,5 mm à 3mm.

## Patentansprüche

1. Vorrichtung (1) zur Injektion eines Produkts, insbesondere zur medizinischen Verwendung, umfassend:
- einen Körper (2), der eine hohle Injektionsnadel (4) aufnimmt, und einen Behälter (10), der ein zu injizierendes Produkt enthält; wobei die Nadel (4) und der Behälter (10) mit Bezug auf den Körper zwischen einer Injektionsposition und einer Rückzugsposition beweglich ist,
- Mittel zum Festhalten der Nadel (5 bis 7; 28, 29), die normalerweise die Nadel (4) in einer Injektionsposition halten, die gelöst werden können, um die Verschiebung der Nadel (4) in die Rückzugsposition freizusetzen;
- Mittel zum Festhalten (39, 41, 45) des Behälters (10), die normalerweise den Behälter (10) in einer Position festhalten, die die Injektion ermöglicht, die gelöst werden können, um die Verschiebung der Nadel (10) in die Rückzugsposition freizusetzen; und
- einen Kolben (11), der im Behälter (10) eingegriffen ist,
- erste Mittel (42) zur Betätigung der Mittel (5 bis 7; 28, 29) zum Festhalten der Nadel (4),
- zweite Mittel (32) zur Betätigung der Mittel (39, 41, 45) zum Festhalten des Behälters (10),
- einen Behälterträger (9), der gleitend auf dem Körper (2) und verschiebbar mit Bezug auf diesen montiert ist, um die Injektion durchzuführen, wobei der Behälter (10) mit diesem Träger (9) verbunden ist und mit Bezug auf diesen zwischen einer Position, die die Injektion ermöglicht, und einer Rückzugsposition beweglich ist,
- Mittel zum Festhalten (3, 47, 48) des Behälterträgers (9), die normalerweise den Behälterträger (9) in Warteposition vor der Injektion festhalten, die gelöst werden können, um die Verschiebung des Behälterträgers (9) freizusetzen und die Injektion zu ermöglichen,
- Mittel zur Betätigung (49) der Lösung der Mittel zum Festhalten (3, 20, 47, 48) des Behälterträgers (9), die die Injektion ermöglichen,
**dadurch gekennzeichnet, dass**:
- die ersten Mittel zur Betätigung verschieden von den zweiten Mitteln zur Betätigung sind, wobei die ersten und die zweiten Mittel zur Betätigung (42, 32) ermöglichen, am Ende der Injektion jeweils die Mittel (5 bis 7; 28, 29) zum Festhalten der Nadel (4) vor dem Lösen der Mittel zum Festhalten (39, 41, 45) des Behälters (10) zu lösen oder gleichzeitig die Mittel (5 bis 7; 28, 29) zum Festhalten der Nadel (4) und die Mittel (39, 41, 45) zum Festhalten des Behälters (10) zu lösen.

2. Vorrichtung (1) nach Anspruch 1, **dadurch gekennzeichnet, dass** die Mittel zum Festhalten (3, 20, 47, 48) des Trägers Folgendes umfassen:
- einen Ring (20), der auf feste Weise im Inneren des Körpers (2) und im proximalen Teil dieses Letzteren montiert ist, wobei dieser Ring (20) an seinem distalen Ende mindestens einen quer liegenden Zahn (48) umfasst,
- mindestens einen Haken (47), der sich am proximalen Ende des Trägers (9) befindet und ausgelegt ist, in den Zahn (48) einzugreifen,
- eine Feder (3) zur Aktivierung der Injektion, deren distales Ende auf einem internen quer liegenden Rücksprung (53) des distalen Endes des Trägers (9) aufliegt, und deren proximales Ende auf einer quer liegenden Wand (54) aufliegt, die sich am distalen Ende des Rings (20) befindet, wobei sich die Feder (3) im komprimierten Zustand befindet, wenn der Zahn (48) in den Haken (47) eingegriffen ist.

3. Vorrichtung (1) nach Anspruch 2, **dadurch gekennzeichnet, dass** die Mittel zur Aktivierung (49) der Lösung der Mittel zum Festhalten des Trägers (9) die Form eines Knopfes (49) aufweisen, der fest mit dem Zahn (48) des Rings (20) verbunden ist, wobei der Knopf (49) an der Außenseite des Körpers (2) über ein Fenster (52) vorspringt, das im Körper (2) zu diesem Zweck angebracht ist.

4. Vorrichtung (1) nach einem beliebigen der vorhergehenden Ansprüche, **dadurch gekennzeichnet, dass** in der Injektionsposition die Nadel (4) eine Eingriffsfläche der Vorrichtung (1) mit der Haut des Patienten auf einer Distanz überragt, die von 0,5 mm bis 3 mm reicht

## Claims

1. A device (1) for injecting a product, in particular a product for medical use, comprising:
- a body (2) receiving a hollow injection needle (4) and a container (10) containing the product to be injected; the needle (4) and the container (10) being movable relative to said body between an injection position and a retraction position;
- needle holding means (5 to 7; 28, 29), holding normally the needle (4) in the injection position, which can be released to free the displacement of the needle (4) to said retraction position;
- container holding means (39, 41, 45), holding normally the container (10) in the position allowing the injection, which can be released to free the displacement of the container (10) to said retraction position; and
- a plunger (11) engaged in the container (10),
- first means (42) for actuating said needle holding means (5 to 7; 28, 29),
- second means (32) for actuating said container holding means (39, 41, 45),
- a container support (9) slidably mounted on the body (2) and displaceable relative to the latter to carry out the injection, said container (10) being linked to this support (9) by being movable relative to the latter between a position allowing the injection and a retraction position,
- means (3, 47, 48) for holding the container support (9), holding normally the container support (9) in the waiting position before the injection, which can be released to free the displacement of the container support (9) and allow the injection,
- means (49) for actuating the release of the means (3, 20, 47, 48) for holding the container support (9) allowing the injection,
**characterized in that**:
- said first actuating means are distinct from said second actuating means, said first and second actuating means (42, 32) allowing, at the end of the injection, to respectively release the needle holding means (5 to 7; 28, 29) before the release of the container holding means (39, 41, 45), or simultaneously release the needle holding means (5 to 7; 28, 29) and the container holding means (39, 41, 45).

2. The device (1) according to claim 1, **characterized in that** the holding means (3, 20, 47, 48) of the support comprise:
- a ring (20), fixedly mounted inside the body (2) and in the proximal portion of the latter, this ring (20) comprising at its distal end at least one transverse tooth (48),
- at least one hook (47) located at the proximal end of the support (9) and intended to be snapped into said tooth (48),
- an injection activation spring (3) whose distal end bears on a transverse inner recess (53) of the distal end of the support (9) and whose proximal end bears on a transverse wall (54) located at the distal end of the ring (20), said spring (3) being in the compressed state when the tooth (48) is snapped into the hook (47).

3. The device (1) according to claim 2, **characterized in that** the means (49) for activating the release of the means for holding the support (9) are in the form of a button (49) secured to the tooth (48) of the ring (20), said button (49) projecting outside the body (2) through a window (52) formed in the body (2) for this purpose.

4. The device (1) according to any one of the preceding claims, **characterized in that**, in the injection position, the needle (4) protrudes from an engagement surface of the device (1) with the skin of the patient, over a distance ranging from 0.5 mm to 3 mm.
